(19) **Europäisches Patentamt**

**European Patent Office**

**Office européen des brevets**

(11) **EP 1 234 814 B1**

(12) **EUROPÄISCHE PATENTSCHRIFT**

(45) Veröffentlichungstag und Bekanntmachung des Hinweises auf die Patenterteilung:
**28.04.2004   Patentblatt 2004/18**

(51) Int Cl.⁷: **C07C 205/12**, C07C 201/08

(21) Anmeldenummer: **02002074.9**

(22) Anmeldetag: **11.02.2002**

(54) **Kontinuierliches adiabatisches Verfahren zur Herstellung von Nitrochlorbenzol**

Continuous adiabatic process for the preparation of nitrochlorobenzene

Procédé continu adiabatique pour la préparation de nitrochlorobenzène

(84) Benannte Vertragsstaaten:
**AT BE CH CY DE DK ES FI FR GB GR IE IT LI LU MC NL PT SE TR**

(30) Priorität: **23.02.2001   DE 10108979**

(43) Veröffentlichungstag der Anmeldung:
**28.08.2002   Patentblatt 2002/35**

(73) Patentinhaber: **Bayer Chemicals AG**
**51368 Leverkusen (DE)**

(72) Erfinder:
 • **Demuth, Ralf, Dr.**
 **40724 Hilden (DE)**
 • **König, Bernd-Michael, Dr.**
 **51467 Bergisch Gladbach (DE)**
 • **Linn, Thomas, Dr.**
 **41517 Grevenbroich (DE)**
 • **Raatz, Hans-Joachim**
 **51381 Leverkusen (DE)**
 • **Weber, Hans-Martin, Dr.**
 **51373 Leverkusen (DE)**
 • **Zirngiebl, Eberhard, Dr.**
 **51061 Köln (DE)**
 • **Leiberich, Ricarda, Dr.**
 **63225 Langen (DE)**

(56) Entgegenhaltungen:
EP-A- 0 675 104          DE-A- 19 808 748
US-A- 4 021 498          US-A- 4 772 757

**Beschreibung**

Die vorliegende Erfindung betrifft ein kontinuierliches adiabatisches Verfahren zur Herstellung von Nitrochlorbenzol, wobei die bei der Durchführung des Verfahrens anfallende Abfallschwefelsäure aufkonzentriert und in die Nitrierreaktion zurückgeführt wird.

Nitrochlorbenzole sind wichtige Zwischenprodukte zur Herstellung von Farbstoffen, Pharmazeutika und Schädlingsbekämpfungsmitteln. Besonders große Nachfrage besteht nach dem bei der Nitrierung von Chlorbenzol zu ca. 35 % anfallenden ortho-Nitrochlorbenzol. Auch für para-Nitrochlorbenzol gibt es einige technische Verwendungen. Weitgehend unerwünscht ist der Anfall von meta-Nitrochlorbenzol, für das nur sehr eingeschränkte technische Verwendungsmöglichkeiten bestehen.

Nitrochlorbenzole werden technisch durch Nitrierung von Chlorbenzol hergestellt. Zur Nitrierung wird üblicherweise eine Mischung aus Schwefelsäure, Salpetersäure und Wasser eingesetzt. Bei der Umsetzung fällt ein erheblicher Anteil mit organischen Verbindungen belasteter Abfallschwefelsäure an, der verfahrens- und kostenintensiv aufgearbeitet werden muss. Um den Anfall von Abfallsäure zu vermeiden, wurden Verfahren entwickelt, die eine Schwefelsäureaufkonzentrierung beinhalten, wobei die Schwefelsäure von Wasser und organischen Verbindungen weitgehend befreit wird und anschließend in einem Kreislaufprozess wieder in die Nitrierreaktion zurückgeführt wird. Besonders elegant gelingt die Aufkonzentrierung der Abfallschwefelsäure bei Durchführung der Nitrierreaktion unter adiabatischen Bedingungen, da hierbei kein Wärmeaustausch mit der Umgebung erfolgt und die bei dem Verfahren frei werdende Energie zu Aufkonzentrierung der Abfallschwefelsäure verwendet werden kann. Zudem können im Gegensatz zu einem wärmeabführenden isothermen Verfahren hochwertigere Werkstoffe verwendet werden, die unter den Reaktionsbedingungen deutlich geringere Korrosionserscheinungen aufweisen.

In US 4,453,027 wird ein adiabatisches Nitrierverfahren zur Herstellung von Mononitrohalogenbenzolen beansprucht. Die verwendete Nitriersäure enthält 11,1 Gew.-% Salpetersäure, 68,5 % Schwefelsäure und 20,4 % Wasser. Für die dabei anfallende Abfallschwefelsäure wird nach der Reaktion eine Temperatur von 100 bis 110°C angegeben. Bei adiabatischer Reaktionsführung beträgt bei der oben genannten Salpetersäurekonzentration die Temperaturerhöhung während der Reaktion ca. 100°C, so dass die Reaktion bei 0 bis 10°C gestartet werden muss und die einzusetzenden Reaktanden bei dieser Temperatur zur Verfügung stehen müssen. Die anfallende Abfallschwefelsäure wird bei Verwendung von 60 %iger Salpetersäure auf 84,1 Gew.-% und bei Verwendung von 98 %iger Salpetersäure immerhin noch auf 77,3 Gew.-% aufkonzentriert. Wird die Reaktionswärme zur Aufkonzentrierung genutzt, so kühlt sich die Abfallschwefelsäure auf ca. 0°C ab. Ein solches Verfahren ist technisch und wirtschaftlich nicht mehr sinnvoll, da Kühlsole und Spezialapparate eingesetzt werden müssen.

In US 4,021,498 ist die adiabatische Mononitrierung von aromatischen Verbindungen beschrieben. Es wird betont, dass die Reaktion unter starkem Rühren durchgeführt werden muss, um einen vollständigen Umsatz zu erreichen. Selbst in einem Rohrreaktor wird ein Rührer benötigt. Allerdings finden sich keine Angaben, wie stark gerührt werden muss, um einen vollständigen Umsatz zu erreichen. Der Fokus des Patents liegt auf der adiabatischen Mononitrierung von Benzol, da sich alle Beispiele darauf beziehen. Konkrete Angaben zur adiabatischen Mononitrierung von halogenierten aromatischen Verbindungen im allgemeinen und Chlorbenzol im besonderen werden nicht gemacht.

In EP 779 270 A wird ein Verfahren zur Herstellung von Mononitroaromaten beschrieben, wobei Reaktoren mit einer Vielzahl spezieller Einbauten, die so angeordnet sind, dass benachbarte Einbauten nahezu senkrecht aufeinanderstehen, verwendet werden. Nachteilig an diesem Verfahren ist, das speziell konzipierte Reaktoren eingesetzt werden müssen, wobei der Erwerb dieser Reaktoren eine kostenintensive Investition darstellt.

In EP 675 104 A ist ein adiabatisches Verfahren zur Nitrierung von Halogenbenzolen beschrieben, wobei die Reaktanden unter Aufwand einer bestimmten Mischenergie vermischt werden und die Vermischung im Temperaturbereich von 60 bis 160°C erfolgt. Mit den genannten Temperaturen lassen sich die für eine adiabatische Fahrweise notwendigen hohen Reaktionsgeschwindigkeiten erzielen. Nachteilig an diesem Verfahren ist aber, dass relativ hohe Anteile an unerwünschtem meta-Nitrochlorbenzol erhalten werden, deren Abtrennung aufwendig und kostenintensiv ist.

Es bestand daher Bedarf an einem Verfahren zur kontinuierlichen Herstellung von Nitrochlorbenzol, welches unter adiabatischen Bedingungen durchgeführt wird und gleichzeitig einen möglichst geringen Anteil an meta-Nitrochlorbenzol liefert.

Überraschenderweise wurde ein Verfahren zur kontinuierlichen Herstellung von Nitrochlorbenzol durch Umsetzung von Chlorbenzol mit Schwefelsäure, Salpetersäure und Wasser gefunden, worin

a) die Einsatzstoffe Chlorbenzol, Schwefelsäure, Salpetersäure und Wasser gleichzeitig oder sukzessive in einen mit Mischungsorganen ausgestatteten Reaktor eingetragen werden und dort so vermischt werden, dass im Reaktor eine mittlere Mischleistungsdichte von 1,5 - 40 Watt/l vorliegt,

b) der Gehalt an Schwefelsäure im Reaktionsgemisch bei der Vermischung, bezogen auf die Summe aus Schwefelsäure, Salpetersäure und Wasser, 70 - 80 Gew.-% beträgt,

c) die Reaktion unter adiabatischen Bedingungen abläuft,

d) am Reaktorausgang eine Trennung des Rohnitrochlorbenzols von der Abfallschwefelsäure erfolgt und

e) die Abfallschwefelsäure auf den ursprünglichen Gehalt an Schwefelsäure aufkonzentriert wird und wieder in die Nitrierreaktion zurückgeführt wird.

dadurch gekennzeichnet, dass
die Temperatur des Reaktionsgemisches bei der Erstvermischung 10 - 50° C beträgt.

Im erfindungsgemäßen Verfahren werden als Einsatzstoffe Chlorbenzol, Schwefelsäure, Salpetersäure und Wasser eingesetzt, wobei Chlorbenzol mit Nitrochlorbenzol versetzt sein kann. Die Menge an Nitrochlorbenzol im Chlorbenzol beträgt in der Regel 0 - 20 Gew.-%. Wasser kann dabei als solches eingesetzt werden oder aber als Verdünnungswasser in der Salpetersäure und/oder der Schwefelsäure in die Reaktion eingebracht werden. Vorzugsweise wird Wasser als Verdünnungswasser in der Salpetersäure und/oder der Schwefelsäure in die Reaktion eingebracht

Die Einsatzstoffe Chlorbenzol, Schwefelsäure, Salpetersäure und Wasser werden im erfindungsgemäßen Verfahren einzeln oder als Mischungen in einen mit Mischungsorganen ausgestatteten Reaktor eingetragen. Der Eintrag der Einsatzstoffe in den Reaktor kann gleichzeitig oder sukzessive erfolgen. Der Eintrag in den Reaktor kann beispielsweise so erfolgen, dass Chlorbenzol und Salpetersäure und gegebenenfalls Wasser als separate Ströme gleichzeitig oder sukzessive der aufkonzentrierten recyclisierten Schwefelsäure zugesetzt werden, wobei die Salpetersäure durch Wasser und/oder Schwefelsäure verdünnt sein kann. Chlorbenzol kann auch mit Wasser und Schwefelsäure vorgemischt werden und die resultierende Mischung wird als separater Strom in den Reaktor eingetragen. Dort erfolgt ein Vermischen mit Salpetersäure, welche mit Schwefelsäure und/oder Wasser vermischt sein kann. Weiterhin können Chlorbenzol und eine Nitriersäure, welche durch Mischen von Schwefelsäure, Salpetersäure und Wasser hergestellt wurde, in separaten Strömen in den Reaktor eingetragen werden. In einer bevorzugten Form des erfindungsgemäßen Verfahrens werden Salpetersäure und die aufkonzentrierte recyclisierte Schwefelsäure zu einer Nitriersäure vermischt, Nitriersäure und Chlorbenzol werden in separaten Strömen in den Reaktor eingetragen. Für das Gelingen der Reaktion ist es von geringerer Bedeutung, in welcher Reihenfolge und Zusammensetzung die Reaktanden in den Reaktor eingetragen werden, solange das Reaktionsgemisch, welches sich nach Vermischung aller Reaktanden ergibt die erfindungsgemäße Zusammensetzung aufweist und die Vermischung in der erfindungsgemäßen Intensität und bei der erfindungsgemäßen Temperatur stattfindet.

Der Gehalt an Schwefelsäure im Reaktionsgemisch zum Zeitpunkt der Vermischung beträgt, bezogen auf die Summe von Schwefelsäure, Salpetersäure und Wasser, 56,5 - 84,5 Gew.-%, bevorzugt 67,1 - 80,9 Gew.-%, besonders bevorzugt 69,5 - 78,6 Gew.-%.

Der Gehalt an Salpetersäure zum Zeitpunkt der Vermischung beträgt, bezogen auf die Summe von Salpetersäure, Schwefelsäure und Wasser, 3 - 10 Gew.-%, bevorzugt 4 - 8 Gew.-%, besonders bevorzugt 4 - 6 Gew.-%. Salpetersäure kann in hochkonzentrierter Form oder in Form von Azeotrop-Salpetersäure, beispielsweise mit einem Gehalt von 60 - 98 Gew.-% Salpetersäure, bevorzugt aber in Form der ca. 60 - 70 Gew.-% aufweisenden und billig verfügbaren, verdünnten Salpetersäure eingesetzt werden.

Im erfindungsgemäßen Verfahren werden alle Reaktanden so vermischt, dass im Reaktor eine mittlere Mischleistungsdichte von 1,5 - 40 Watt/l, bevorzugt 1,5 - 30 Watt/l, vorliegt. Zur Vermischung können die in der Technik bekannten Mischorgane wie beispielsweise statische Mischer, Pumpen, Düsen, Rührer oder Kombinationen hiervon eingesetzt werden. Die Bestimmung der Mischleistungsdichte, ausgedrückt in Watt pro Liter, in einem kontinuierlich betriebenen Reaktor wird folgendermaßen durchgeführt:

$$\text{Mischleistungsdichte} = \text{Leistung } P \ / \ \text{Volumen } V \ [\text{W/l}]$$

$$P = \text{Durchsatz der Reaktanden } [\text{m}^3/\text{s}] \times \text{dynamischem Druckabfall } \Delta p_{dyn} \ [\text{N/m}^2]$$

$$\Delta p_{dyn} = \text{gesamter Druckabfall } \Delta p_{ges} - \text{statischer Druckabfall } \Delta p_{stat}$$

Da die mittlere Mischleistungsdichte auf jeden Liter der Reaktionsmischung einwirkt und diese Reaktionsmischung erst im Reaktor vorliegt, wird bei der Berechnung der mittleren Mischleistungsdichte als Volumen V das Volumen des Reaktors eingesetzt, in welchem die Reaktion durchgeführt wird.

Die Temperatur des Reaktionsgemisches bei der Erstvermischung beträgt 10 - 50°C, bevorzugt 20 - 50°C und besonders bevorzugt 30 - 45°C. Von der Temperatur des Reaktionsgemisches bei der Vermischung und vom Umsatz ist die Endtemperatur abhängig, sie übersteigt im allgemeinen 130°C nicht, vorzugsweise liegt sie unterhalb von 100°C.

Vorzugsweise sollte die Reaktion möglichst frei von Rückvermischung ablaufen, was beispielsweise durch Dispergierungen der Reaktionsmischung erreicht werden kann. Dies wird vorzugsweise durch im Reaktor für diesen Zweck bestimmte Einbauten oder Organe wie beispielsweise Lochbleche, Schlitzbleche, Prallbleche, Strömungsbrecher, Umlenkbleche, Statikmischer oder Rührer bewerkstelligt.

Als kontinuierlich betriebene Reaktoren für das erfindungsgemäße Verfahren seien beispielsweise genannt: Rohrreaktoren, vorzugsweise mit Einbauten zur Dispergierung wie beispielsweise Lochblechen, Schlitzblechen, Prallblechen, Strömungsbrechern, Umlenkblechen, Statikmischern, Rührern und Ähnlichem, stark gerührte Kessel in Kaskadenanordnung, Schlaufenreaktoren mit wie oben beschriebenen Einbauten, Kombinationen mehrerer der genannten Apparate, weitere gleichwirkende Reaktoren, wie Kammerreaktoren mit Rührern in jeder Kammer. In bevorzugter Weise werden im erfindungsgemäßen Verfahren Rohrreaktoren mit Einbauten eingesetzt. Die Einbauten sind bevorzugt Lochbleche. Alle Einbauten stellen Unterteilungen der Gesamtapparatur dar, die gleichermaßen der Dispergierung und der weitgehenden Verhinderung der Rückvermischung dienen.

Nach dem intensiven Vermischen, nach jeder Dispergierung und nach dem Durchströmen einer gewissen Teillänge des Reaktors wird ein Koaleszieren der Dispersionströpfchen beobachtet, was durch Redispergierung rückgängig gemacht wird. Die Zahl der Dispergierungsvorgänge beträgt vorzugsweise 2 - 50, bevorzugt 3 - 30, besonders bevorzugt 4 - 20. Die bei der Vermischung der Reaktanden auf jeden Liter des Reaktionsgemisches einwirkende erfindungsgemäße mittlere Mischleistungsdichte von 1,5 - 40 Watt/l wird vorzugsweise zur Überwindung der bei den Dispergierungsvorgängen auftretenden Druckverluste eingesetzt.

Im erfindungsgemäßen Verfahren wird Chlorbenzol formelmäßig mit Salpetersäure zu Nitrochlorbenzol und Wasser umgesetzt. Somit werden Chlorbenzol und Salpetersäure in das Verfahren eingeführt und Nitrochlorbenzol und Wasser ausgeschleust, während das beschriebene Schwefelsäure/Wassergemisch das Reaktionsmedium darstellt. Da bei der technischen Realisierung vorteilhafterweise verdünnte Salpetersäuren eingesetzt werden, muss zusätzlich zum Reaktionswasser auch noch Verdünnungswasser der Salpetersäure ausgeschleust werden.

Im erfindungsgemäßen Verfahren findet am Reaktorausgang eine Trennung des Rohnitrochlorbenzols von der Abfallschwefelsäure statt. Die Abtrennung kann in dem Fachmann bekannten Apparaturen oder mit Hilfe von dem Fachmann bekannten Mitteln erreicht werden.

So kann eine Trennung beispielsweise durch Einsatz eines statischen Scheiders erfolgen. Die so erhaltene Abfallschwefelsäure ist weitgehend salpetersäurefrei und enthält zum überwiegenden Teil Schwefelsäure und Wasser, gegebenenfalls sind zu einem geringen Anteil organische Verunreinigungen und/oder Nitrosylschwefelsäure enthalten. Die Schwefelsäurekonzentration der Abfall-schwefelsäure beträgt erfindungsgemäß 70 - 85 Gew.-%, bevorzugt 72 - 80 Gew.-%, besonders bevorzugt 76 - 79 Gew.-%.

Zum Wiedereinsatz wird die Abfallschwefelsäure erfindungsgemäß auf den ursprünglichen Gehalt an Schwefelsäure aufkonzentriert und in die Nitrierreaktion zurückgeführt. Bei der Aufkonzentrierung wird Wasser (Reaktionswasser und gegebenenfalls Verdünnungswasser) destillativ ausgeschleust. Hierzu wird in bevorzugter Weise die von der Abfallschwefelsäure aufgenommene Reaktionswärme ausgenutzt.

Die Aufkonzentrierung wird vorzugsweise in einem Verdampfer durchgeführt, welcher vorzugsweise bei einem Druck von 60 - 200 mbar, besonders bevorzugt von 60 - 180 mbar und ganz besonders bevorzugt von 80 - 150 mbar betrieben wird. Die Temperatur der Abfallschwefelsäure im Austritt des Verdampfers beträgt dabei vorzugsweise 100 - 200°C, besonders bevorzugt 130 - 190°C und ganz besonders bevorzugt 145 - 165°C. Die Temperatur der ablaufenden aufkonzentrierten Abfallschwefelsäure wird vorzugsweise dazu genutzt, um in einem Gegenstromwärmetauscher die in den Verdampfer strömende Abfallschwefelsäure aufzuheizen.

Die Aufkonzentrierung wird vorzugsweise in einem einstufigen Verfahren durchgeführt, wobei vorzugsweise ein kommerziell erhältlicher, kaskadierter Verdampfer mit Tantalrohrbündel verwendet wird, bei dem vom Eintritt kommend mit jeder Kaskade die Säurekonzentration erhöht wird, so dass in den ersten Kaskaden eine relativ niederkonzentrierte Säure vorliegt. Vorteil der niedrigen Konzentration in der ersten Kaskade ist einerseits, dass der Siedepunkt noch niedrig ist und somit eine hohe treibende Temperaturdifferenz für den Wärmeübergang vorliegt (kleiner Verdampfer) und andererseits, dass bei niedrigen Säurekonzentration eventuell in der Abfallsäure vorliegende Nitrosylschwefelsäure aus der Reaktion besser entfernbar ist. Somit wird durch die Verwendung eines kaskadierten Verdampfers im erfindungsgemäßen Verfahren das üblicherweise durchgeführte Ausblasen der Nitrosylschwefelsäure mit Schwefeldioxid und somit ein zusätzlicher Verfahrensschritt vermieden. Um Ablagerungen von organischen Verbindungen, insbesondere von Nitrochlorbenzol, auf dem Kondensator zu verhindern, wird dieser in einer bevorzugten Ausführungsform kontinuierlich mit Chlorbenzol berieselt. Die ablaufende organische Phase aus Chlorbenzol und Nitrochlorbenzol kann als Einsatzstoff im erfindungsgemäßen Verfahren eingesetzt werden.

## Beispiele

### Beispiel 1

In einen Rohrreaktor wurden kontinuierlich 186 kg/h 81 %ige Schwefelsäure, 13,3 kg/h 70,6 %ige Salpetersäure und 19,7 kg/h Chlorbenzol, welches ca. 7 - 8 % Nitrochlorbenzol enthielt, zugeführt. Die mittlere Mischleistungsdichte betrug 6,2 Watt/l. Die Temperatur bei der Vermischung betrug 40°C. Nach ca. 180 sec. hatte das Reaktionsgemisch das Reaktorende erreicht, die Temperatur am Reaktorausgang betrug 92 - 94°C. Das Reaktionsgemisch wurde auf ca. 120°C erwärmt, die Phasenscheidung erfolgte innerhalb von 90 Sekunden in einem statischen Scheider. Die Abfallschwefelsäure wurde auf den ursprünglichen Gehalt an Schwefelsäure aufkonzentriert und wieder in die Nitrierreaktion zurückgeführt. Der zur Kondensation des Wassers verwendete Kondensator wurde mit Chlorbenzol berieselt, um Ablagerungen von eventuell in der Abfallschwefelsäure gelöstem Nitrochlorbenzol zu verhindern. Die Rohnitrochlorbenzolphase wurde gaschromato-graphisch analysiert und wies folgende Zusammensetzung auf:

| Chlorbenzol | 8,97 |
|---|---|
| ortho-Nitrochlorbenzol | 34,14 |
| meta-Nitrochlorbenzol | 0,87 |
| para-Nitrochlorbenzol | 55,83 |
| Dinitroverbindungen | 0,19 |

### Beispiel 2 (nicht erfindungsgemäß)

In einen Rohrreaktor wurden kontinuierlich 186 kg/h 75 %ige Schwefelsäure, 9,6 kg/h, 65 %ige Salpetersäure und 12,3 kg/h Chlorbenzol, welches ca. 7 % Nitrochlorbenzol enthielt, eingetragen. Die mittlere Mischleistungsdichte betrug 6,2 Watt/l. Die Temperatur bei der Vermischung betrug ca. 110°C. Nach ca. 180 Sekunden hatte das Reaktionsgemisch das Reaktorende erreicht, die Temperatur am Reaktorausgang betrug 140°C. Die Phasenscheidung erfolgte in einem statischen Scheider. Die Abfallschwefelsäure wurde auf den ursprünglichen Gehalt an Schwefelsäure aufkonzentriert und wieder in die Nitrierreaktion zurückgeführt. Der zur Kondensation des Wassers verwendete Kondensator wurde mit Chlorbenzol berieselt, um Ablagerungen von eventuell in der Abfallschwefelsäure gelöstem Nitrochlorbenzol zu verhindern. Die Rohnitrochlorbenzolphase wurde gaschromato-graphisch analysiert und wies folgende Zusammensetzung auf:

| Chlorbenzol | 2,70 |
|---|---|
| ortho-Nitrochlorbenzol | 37,64 |
| meta-Nitrochlorbenzol | 1,56 |
| para-Nitrochlorbenzol | 57,83 |
| Dinitroverbindungen | 0,26 |

## Patentansprüche

1. Verfahren zur kontinuierlichen Herstellung von Nitrochlorbenzol durch Umsetzung von Chlorbenzol mit Schwefelsäure, Salpetersäure und Wasser,
worin

   a) die Einsatzstoffe Chlorbenzol, Schwefelsäure, Salpetersäure und Wasser gleichzeitig oder sukzessive in einen mit Mischungsorganen ausgestatteten Reaktor eingetragen werden und so vermischt werden, dass im Reaktor eine mittlere Mischleistungsdichte von 1,5 - 40 Watt/l vorliegt,

   b) der Gehalt an Schwefelsäure im Reaktionsgemisch bei der Vermischung, bezogen auf die Summe aus Schwefelsäure, Salpetersäure und Wasser, 70 - 80 Gew.-% beträgt,

   c) die Reaktion unter adiabatischen Bedingungen abläuft,

   d) am Reaktorausgang eine Trennung des Rohnitrochlorbenzols von der Abfallschwefelsäure erfolgt und

e) die Abfallschwefelsäure auf den ursprünglichen Gehalt an Schwefelsäure aufkonzentriert wird und wieder in die Nitrierreaktion zurückgeführt wird.

**dadurch gekennzeichnet, dass**

die Temperatur des Reaktionsgemisches bei der Erstvermischung 10 - 50°C beträgt.

2. Verfahren gemäß Anspruch 1, **dadurch gekennzeichnet, dass** der Gehalt an Salpetersäure im Reaktionsgemisch bei der Vermischung, bezogen auf die Summe aus Schwefelsäure, Salpetersäure und Wasser 3 - 10 Gew.-% beträgt.

3. Verfahren gemäß einem oder mehreren der vorangegangenen Ansprüche 1 und 2, **dadurch gekennzeichnet, dass** Salpetersäure in Form einer 60 - 70 %igen Salpetersäure eingesetzt wird.

4. Verfahren gemäß einem oder mehreren der vorangegangenen Ansprüche 1 bis 3, **dadurch gekennzeichnet, dass** Chlorbenzol in einer Menge von 1 1,3 Äquivalenten pro Äquivalent Salpetersäure eingesetzt wird.

5. Verfahren gemäß einem oder mehreren der vorangegangenen Ansprüche 1 bis 4, **dadurch gekennzeichnet, dass** die mittlere Mischleistungsdichte 1,5 - 30 Watt/l beträgt.

6. Verfahren gemäß einem oder mehreren der vorangegangenen Ansprüche 1 bis 5, **dadurch gekennzeichnet, dass** die Temperatur des Reaktionsgemisches bei der Erstvermischung 20 - 50°C beträgt.

7. Verfahren gemäß einem oder mehreren der vorangegangenen Ansprüche 1 bis 6, **dadurch gekennzeichnet, dass** die Aufkonzentrierung der Abfallschwefelsäure in einem Verdampfer durchgeführt wird.

8. Verfahren gemäß einem oder mehreren der vorangegangenen Ansprüche 1 bis 7, **dadurch gekennzeichnet, dass** die Aufkonzentrierung der Abfallschwefelsäure in einem Verdampfer bei einem Druck von 60 - 200 mbar durchgeführt wird.

9. Verfahren gemäß einem oder mehreren der vorangegangenen Ansprüche 1 bis 7, **dadurch gekennzeichnet, dass** die Temperatur der Abfallschwefelsäure im Austritt des Verdampfers 100 - 200°C beträgt.

10. Verfahren gemäß einem oder mehreren der vorangegangenen Ansprüche 7 bis 9, **dadurch gekennzeichnet, dass** es sich bei dem Verdampfer um einen kaskadierten Verdampfer mit Tantalrohrbündel handelt

**Claims**

1. Process for the continuous preparation of nitrochlorobenzene by reacting chlorobenzene with sulphuric acid, nitric acid and water, in which

    a) the feed stocks chlorobenzene, sulphuric acid, nitric acid and water are introduced simultaneously or successively into a reactor equipped with mixing elements and are mixed such that in the reactor an average mixing power density of 1.5-40 watt/l is present,

    b) the content of sulphuric acid in the reaction mixture during mixing, based on the sum of sulphuric acid, nitric acid and water, is 70-80% by weight,

    c) the reaction proceeds under adiabatic conditions,

    d) at the reactor outlet, the crude nitrochlorobenzene is separated from the waste sulphuric acid and

    e) the waste sulphuric acid is reconcentrated to the original content of sulphuric acid and recycled into the nitration reaction,

**characterized in that**

the temperature of the reaction mixture during initial mixing is 10-50°C.

**2.** Process according to Claim 1, **characterized in that** the content of nitric acid in the reaction mixture during mixing, based on the sum of sulphuric acid, nitric acid and water, is 3-10% by weight.

**3.** Process according to one or more of the preceding Claims 1 and 2, **characterized in that** the nitric acid is employed in the form of a 60-70% strength nitric acid.

**4.** Process according to one or more of the preceding Claims 1 to 3, **characterized in that** the chlorobenzene is employed in an amount of 1-1.3 equivalents per equivalent of nitric acid.

**5.** Process according to one or more of the preceding Claims 1 to 4, **characterized in that** the average mixing power density is 1.5-30 watt/l.

**6.** Process according to one or more of the preceding Claims I to 5, **characterized in that** the temperature of the reaction mixture during initial mixing is 20-50°C.

**7.** Process according to one or more of the preceding Claims 1 to 6, **characterized in that** the reconcentration of the waste sulphuric acid is carried out in an evaporator.

**8.** Process according to one or more of the preceding Claims 1 to 7, **characterized in that** the reconcentration of the waste sulphuric acid is carried out in an evaporator at a pressure of 60-200 mbar.

**9.** Process according to one or more of the preceding Claims 1 to 7, **characterized in that** the temperature of the waste sulphuric acid in the evaporator outlet is 100-200°C.

**10.** Process according to one or more of the preceding Claims 7 to 9, **characterized in that** the evaporator is a cascade evaporator with tantalum tube bundle.

## Revendications

**1.** Procédé de préparation en continu de nitrochlorobenzène comprenant la réaction de chlorobenzène avec de l'acide sulfurique, de l'acide nitrique, et de l'eau, dans lequel

(a) on introduit simultanément ou successivement les alimentations en chlorobenzène, acide sulfurique, acide nitrique, et eau dans un réacteur équipé d'organes de mélange et on les mélange à une densité de puissance de mélange moyenne de 1,5 à 40 watts/l,
(b) la quantité d'acide sulfurique dans le mélange réactionnel pendant le mélange, rapporté à la somme de l'acide sulfurique, l'acide nitrique, et l'eau, est de 70 à 80 % en poids,
(c) la réaction a lieu dans des conditions adiabatiques,
(d) à la sortie du réacteur, on sépare le nitrochlorobenzène brut de l'acide sulfurique usé, et
(e) on reconcentre l'acide sulfurique usé à la quantité d'origine d'acide sulfurique et on le recycle dans la réaction de nitration, **caractérisé en ce que** la température de mélange réactionnel pendant le mélange initial est de 10 à 50°C.

**2.** Procédé selon la revendication 1, **caractérisé en ce que** la quantité d'acide nitrique dans le mélange réactionnel pendant le mélange, rapporté à la somme de l'acide sulfurique, l'acide nitrique, et l'eau, est 3 à 10 % en poids.

**3.** Procédé selon l'une ou plusieurs des revendications 1 et 2, **caractérisé en ce que** l'acide nitrique est employé sous la forme d'acide nitrique à 60 à 70 %.

**4.** Procédé selon l'une ou plusieurs des revendications 1 à 3, **caractérisé en ce qu'**on emploie 1 à 1,3 équivalents de chlorobenzène par équivalent d'acide nitrique.

**5.** Procédé selon l'une ou plusieurs des revendications 1 à 4, **caractérisé en ce que** la densité de puissance de mélange moyenne est de 1,5 à 30 watts/litre.

**6.** Procédé selon l'une ou plusieurs des revendications 1 à 5, **caractérisé en ce que** la température du mélange réactionnel pendant le mélange initial des 20 à 50°C.

**7.** Procédé selon l'une ou plusieurs des revendications 1 à 6, **caractérisé en ce qu'**on effectue la reconcentration de l'acide sulfurique usé dans un évaporateur.

**8.** Procédé selon l'une ou plusieurs des revendications 1 à 7, **caractérisé en ce qu'**on effectue la reconcentration de l'acide sulfurique usé dans un évaporateur à une pression de 60 à 200 mbar.

**9.** Procédé selon l'une ou plusieurs des revendications 1 à 7, **caractérisé en ce que** la température de l'acide sulfurique à la sortie de l'évaporateur est de 100 à 200°C.

**10.** Procédé selon l'une ou plusieurs des revendications 7 à 9, **caractérisé en ce que** l'évaporateur est un évaporateur à cascade avec un paquet de tubes de tantale.